(19) Europäisches Patentamt
European Patent Office
Office européen des brevets

(11) Veröffentlichungsnummer: **0 494 456 A1**

(12) # EUROPÄISCHE PATENTANMELDUNG

(21) Anmeldenummer: **91122367.5**

(22) Anmeldetag: **30.12.91**

(51) Int. Cl.⁵: **C07D 239/42**, C07D 251/44, C09B 62/028

(30) Priorität: **10.01.91 DE 4100513**

(43) Veröffentlichungstag der Anmeldung: **15.07.92 Patentblatt 92/29**

(84) Benannte Vertragsstaaten: **CH DE FR GB LI**

(71) Anmelder: **BAYER AG**

**W-5090 Leverkusen 1 Bayerwerk(DE)**

(72) Erfinder: **Jäger, Horst, Dr.**
**Carl-Rumpff-Strasse 37**
**W-5090 Leverkusen 11(DE)**
Erfinder: **Arnold, Siegbert, Dr.**
**Lotharstrasse 18**
**W-5300 Bonn(DE)**
Erfinder: **Sommer, Richard, Dr.**
**Ackerstrasse 51**
**W-5068 Odenthal-Glöbusch(DE)**

(54) **Verbessertes Verfahren zur Herstellung eines substituierten 2-Amino-1-sulfonaphthalins.**

(57) 2,4,6-Trifluor-5-chlor-pyrimidin und die wäßrige Lösung eines Salzes der 2-Amino-5-aminomethyl-naphthalin-1-sulfonsäure werden gleichzeitig in etwa äquimolaren Mengen in einen Reaktor eindosiert und ergeben ein wertvolles Zwischenprodukt zur Herstellung hochreiner Farbstoffe.

EP 0 494 456 A1

Die Erfindung betrifft ein Verfahren zur Herstellung eines 2-Amino-1-sulfonaphthalins, welches mit einer Reaktivgruppe substituiert ist und die Verwendung der so erhaltenen Substanzen zur Herstellung eines Reaktivfarbstoffes.

Aus der DE-A 2 264 698 sind bereits Farbstoffe auf Basis von 1-Sulfo-2-amino-naphthalin bekannt, in welche nach der Diazotierung und Kupplung Reaktivgruppen eingeführt werden. Diese Herstellung ist insbesondere hinsichtlich der Reinheit der entsprechenden Endprodukte verbesserungswürdig. Aus der DE-A 3 223 257 und der DE-A 2 831 912 ist als Diazokomponente bereits 2-Amino-5-(2',4'-difluor-5-'-chlor-pyrimidinyl-6')-amino-methyl-naphthalin-1-sulfonsäurebekannt.

Die Erfindung betrifft ein verbessertes Verfahren zur Herstellung von Verbindungen der Formel

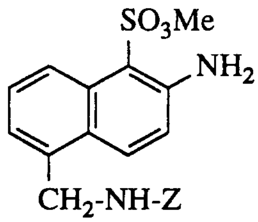

(1),

worin

Z = faserreaktiver Rest einer Reaktivkomponente, der mindestens ein reaktives Fluoratom enthält, insbesondere Rest der Pyrimidin- oder Aminotriazinreihe und

Me = Kation, insbesondere Li, Na, K, H dadurch gekennzeichnet, daß man eine fluorhaltige Reaktivkomponente der Formel

Z-F (2) (F = Fluor)

und eine wässrige Lösung eines Salzes, insbesondere eines Alkalisalzes, der 2-Amino-5-amino-methyl-naphthalin-1-sulfonsäure (3) in einen Reaktor dosiert, wobei die Reaktivkomponente (2) und die Sulfonsäure (3) gleichzeitig in den Reaktor unter Reaktion eindosiert werden.

Vorzugsweise werden die Reaktanden im Molverhältnis von etwa 1:1 bei Temperaturen von -5 ° C bis 50 ° C, wobei sich die Temperatur nach der Reaktivität der Reaktivkomponenten (2) richtet und in einem pH-Bereich von 8-11, vorzugsweise 9-10, insbesondere 9,3-9,7, gleichzeitig zusammen dosiert.

Dies kann so geschehen, daß kein Vorlauf der Reaktanden (2) und (3) erfolgt. Es ist jedoch zweckmäßig, daß man (2) mit einem Vorlauf von 1-20 %, insbesondere 5-15 %, bezogen auf die Gesamtmenge (2) vorlegt und den Rest gleichmäßig mit der Lösung von (3) zudosiert.

Das Verfahren kann kontinuierlich oder diskontinuierlich ausgeführt werden. Bei der diskontinuierlichen Arbeitsweise verfährt man so, daß man in eine wässrige Vorlage, eine wässrige Lösung eines Alkalisalzes (3), eine Reaktivkomponente (2) und gegebenenfalls Alkali, das zur Einhaltung des angegebenen pH-Bereiches dient, eindosiert. Das Molverhältnis von (2) zu (3) beträgt im allgemeinen 1,2-1,0:1,0, insbesondere 1,1-1,0:1.

Vorzugsweise führt man die Reaktion so aus, daß die wässrige Vorlage einen Puffer enthält, der für den pH-Bereich von 8-11 geeignet ist. Die Menge des Puffers sollte so beschaffen sein, daß eine ausreichende Pufferung gewährleistet ist. Pro Mol Alkalisalz (3) verwendet man 0,1-2,0 Mol Puffer, Geeignete Puffersysteme sind Phosphate, Carbonate, Borate. Vorzugsweise verwendet man Alkaliborate.

Als Alkali zur Aufrechterhaltung des pH-Bereiches kann man wässrige Lösungen der Alkalihydroxyde wie Lithium-, Natrium- oder Kaliumhydroxid verwenden. Man kann aber auch soviel Puffersubstanz vorlegen, daß eine Korrektur des angegebenen pH-Bereiches während der Acylierung durch Alkali nicht erforderlich ist.

Beispiele für Reaktivkomponenten (2) sind:

Aminotriazinreihe:

2,4-Difluor-6-(o-, m-, p-sulfophenyl)-amino-triazin, 2,4-Difluor-6-methoxytriazin, 2,4-Difluor-6-(2', 5'-disulfophenyl)-amino-triazin, 2,4-Difluor-6-(6'-sulfonaphthyl-2')-amino-triazin, 2,4-Difluor-6-(2'-methyl-5'-sulfophenyl)-amino-triazin, 2,4-Difluor-6-(2'-methyl-4'-sulfophenyl)-amino-triazin, 2,4-Difluor-6-(2'-chlor-4'-sulfophenyl)-amino-triazin, 2,4-Difluor-6-(2'-methoxy-4'-sulfophenyl)-amino-triazin, 2,4-Difluor-6-(o-, m-, p-carboxyphenyl)-amino-triazin, 2,4-Difluor-6-(1'-sulfonaphthyl-(2'))-amino-triazin, 2,4-Difluor-6-aminotriazin, 2,4-Difluor-6-methylaminotriazin, 2,4-Difluor-6-ethylaminotriazin, 2,4-Difluor-6-methoxyethoxytriazin, 2,4-Difluor-6-$\beta$-methoxy-ethylaminotriazin.

Pyrimidinreihe:

2,4-Difluor-6-methylpyrimidin, 2,6-Difluor-4-methyl-5-chlor-pyrimidin, 2,4-Difluor-pyrimidin-5-ethylsulfon, 2,6-Difluor-4-chlor-pyrimidin, 2,4,6-Trifluor-5-chlor-pyrimidin, 2,6-Difluor-4-methyl-5-brompyrimidin, 2,6-Difluor-5,6-dichlor- oder -dibrompyrimidin, 4,6-Difluor-2,5-dichlor-oder -dibrompyrimidin, 2,6-Difluorpyrimidin, 2,4,6-Trifluor-5-chlor-methylpyrimidin, 2,6-Difluor-5-methyl-4-chlorpyrimidin, 2,6-Difluor-5-chlorpyrimidin, 2,4,6-Trifluor-5-methylpyrimidin, 2,4-Difluor-5-cyan-pyrimidin, 2,4-Difluor-5-methyl-pyrimidin, 2,4-Difluor-5-trifluormethylpyrimidin, 2,4-Difluor-5-methylsulfonyl-pyrimidin, 2,4-Difluor-5-phenylpyrimidin, 4,6-Difluor-2-methyl-pyrimidin, 4,6-Difluor-6-chlor-2-methylpyrimidin, 4,5,6-Trifluorpyrimidin, 4,6-Difluor-6-chlor-2-trifluormethyl-pyrimidin, 4,6-Difluor-2-phenylpyrimidin, 4,6-Difluor-5-cyan-pyrimidin, 4,6-Difluor-5-nitropyrimidin, 4,6-Difluor-5-methylsulfonylpyrimidin, 4,6-Difluor-5-phenylsulfonylpyrimidin, 4,6-Difluor-5-phenylsulfonylpyrimidin, 4,6-Difluor-5-chlor-pyrimidin.

Ganz besonders bevorzugt ist ein verbessertes Verfahren zur Herstellung einer Verbindung der Formel

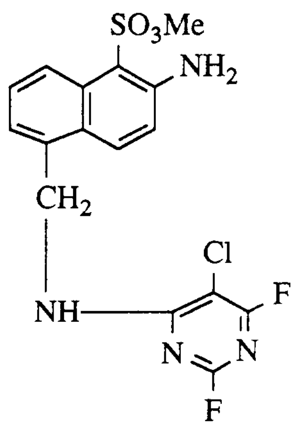

(4),

worin

Me = Kation, insbesondere Li, Na, K, H,

dadurch gekennzeichnet, daß man 2,4,6-Trifluor-5-chlor-pyrimidin und eine wässrige Lösung eines Salzes, insbesondere eines Alkalisalzes, der 2-Amino-5-amino-methyl-naphthalin-1-sulfonsäure (3) in einem Reaktor dosiert, wobei das Pyrimidin und die Sulfonsäure (3), gleichzeitig in den Reaktor unter Reaktion eindosiert werden. Vorzugsweise werden die Reaktanden im Molverhältnis von etwa 1:1 bei Temperaturen von -5 °C bis 20 °C, vorzugsweise bei -2 °C bis 5 °C und in einem pH-Bereich von 8-11, vorzugsweise 9-10, insbesondere 9,3-9,7 gleichzeitig zusammen dosiert.

Gegenstand der Erfindung ist weiterhin ein Verfahren zur Herstellung der Farbstoffe der Formel

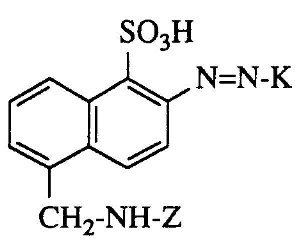

(5),

worin

K den Rest einer Kupplungskomponente HK (6) bedeutet,

dadurch gekennzeichnet, daß man die vorzugsweise in wässriger Anschlämmung vorliegenden, erfindungsgemäß hergestellten Amine (1) diazotiert und auf Kupplungskomponente (6) kuppelt.

Ganz besonders bevorzugt ist ein Verfahren zur Herstellung von Farbstoffen der Formel

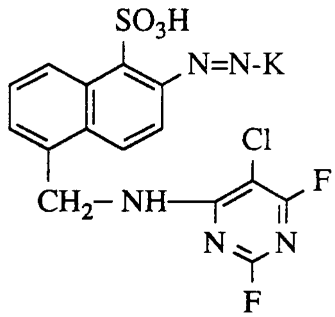

(7),

worin

K den Rest einer Kupplungskomponente HK (6) bedeutet,

dadurch gekennzeichnet, daß man das vorzugsweise in wässrige Anschlämmung vorliegende erfindungsgemäß hergestellte Amin (4) diazotiert und auf Kupplungskomponenten (6) kuppelt.

Vorzugsweise wird dieses Verfahren so durchgeführt, daß man in Gegenwart eines anionischen Dispergiermittels diazotiert. Dieses wird im allgemeinen in Mengen von 20 bis 200 g, insbesondere von 70 bis 150 g pro Mol des zu diazotierenden Amins (1), eingesetzt. Anionische Dispergiermittel sind Ligninsulfonate und Kondensationsprodukte aus aromatischen Sulfonsäuren mit Formaldehyd. Insbesondere verwendet man die Natriumsalze der Kondensationsprodukte aus Naphthalinsulfonsäure mit Formaldehyd.

Die Diazotierung wird in Wasser nach der direkten oder indirekten Methode durchgeführt (siehe Houben-Weyl Band 10, Teil 3,S.16-22; 4.Auflage).

Das Nitrit kann in Form einer wässrigen Natrium- oder Lithium-Nitritlösung zugegeben werden.

Bei indirekter Arbeitsweise wird das Amin (1) in Wasser neutral gelöst, mit der stöchiometrischen Menge eines Nitrits versetzt und dann dieses Gemisch in eine Vorlage aus Wasser und Säure gegeben.

Bei der direkten Diazotierung wird die Suspension des Amins (1) in Wasser nach Zusatz einer Säure allmählich mit der berechneten Menge eines Nitrits versetzt bis der Endpunkt der Reaktion erreicht ist.

Die Farbstoffe können durch Zugabe von Salz isoliert und getrocknet werden. Ebenso gut können aus den Herstellungs-Lösungen oder Suspensionen konzentrierte wässrige Lösungen hergestellt werden, in dem man diese Lösungen oder Suspensionen in Wasser einer Entsalzung, z.B. durch Druckpermeation unterwirft. Bevorzugt werden die Herstellungs-Lösungen oder Suspensionen, gegebenenfalls nach einer Druckpermeation direkt zur Trockne gebracht.

So besteht ein weiterer Gegenstand der Erfindung darin, daß man die nach der Kupplung anfallenden wässrigen Lösungen oder Suspensionen der Farbstoffe (5) ohne Zwischenisolierung direkt zur Trockene bringt, gegebenenfalls nach Zusatz der für Reaktivfarbstoffe üblichen Stellmittel. Dies kann durch Eindampfen geschehen. Man kann die Herstellunsgemische auch über einen Walzentrockner führen. Bevorzugt wird der Farbstoff durch Sprühtrocknen isoliert.

Kupplungskomponenten HK (6) sind vor allem geeignete Verbindungen der Aminobenzol- und Naphthalinreihe, z.B. Aniline, N-mono-substituierte Aniline, m-Phenylendiaminderivate, Aminonaphthaline, Aminonaphthalinsulfonsäuren, Naphtholsulfonsäuren oder Aminonaphtholsulfonsäuren, ferner Pyrazolone, Aminopyrazole, Aminopyridine, Hydroxypyridine/pyridone, Hydroxypyrimidine, Indole, Barbitursäurederivate oder Acetoacetarylide.

Bevorzugte Kupplungskomponenten HK sind Verbindungen der Formel

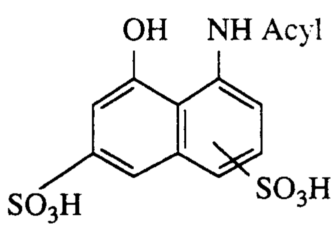

(7),

worin

Acyl = Acylgruppe, wie Alkylcarbonyl, Alkylsulfonyl, Arylcarbonyl oder Arylsulfonyl, wobei Alkyl bevorzugt für gegebenenfalls durch $C_1$-$C_4$-Alkoxy, Cl, COOH, $SO_3H$ substituiertes $C_1$-$C_4$-Alkyl und Aryl bevorzugt für gegebenenfalls durch $C_1$-$C_4$-Alkyl, $C_1$-$C_4$-Alkoxy, Cl substituier-

tes Phenyl steht.

Insbesondere bedeutet Acyl Benzoyl.

Die Formeln der wasserlöslichen Verbindungen in der Beschreibung und in den Beispielen sind die der freien Säuren. Isoliert und angewandt werden die Stoffe im allgemeinen in Form ihrer Alkalisalze, insbesondere der Lithium-, Natrium- oder Kaliumsalze.

Beispiel 1

Acylierung

252 g 2-Amino-5-aminomethyl-naphthalin-1-sulfonsäure (1,0 Mol; liegt als Betain vor) werden in 1 800 ml Wasser durch Zugabe von 1 Mol Natronlauge gelöst. In einer Vorlage werden in 400 ml Wasser 38 g Borsäure (0,61 Mol) mit 0,38 mol Natronlauge bei pH 9,5 gelöst. Anschließend fügt man 1 kg Eis hinzu. In diese Vorlage werden gleichzeitig unter gutem Rühren 177 g 2,4,6-Trifluor-5-chlor-pyrimidin und die alkalische Lösung der 2-Amino-5-aminomethyl-naphthalin-1-sulfonsäure in 20 Minuten eindosiert. Durch Einwerfen von Eis hält man die Temperatur bei 0 °C. Zur Einhaltung des pH-Bereiches von 9,5 (9,3 bis 9,7) müssen während der Acylierung ca. 0,9 Mol Natronlauge zugeführt werden. Man rührt noch ca. 1 Stunde nach, wobei der pH noch geringfügig korrigiert werden muß. Das Kondensationsprodukt ist zum Teil ausgefallen. Volumen 6 l.

Diazotierung Kupplung Isolierung

Diese Suspension wird mit 120 g eines anionischen Dispergiermittels (Na-Salz des Kondensationsproduktes aus Naphthalinsulfonsäure und Formaldehyd) versetzt und dann in bekannter Weise direkt diazotiert. Die Menge der Kupplungskomponente richtet sich nach dem Nitritverbrauch bei der direkten Diazotierung. Diese Menge an 1-Hydroxy-8-benzoylamino-naphthalin-3,5-disulfonsäure wird in ca. 1 500 ml Wasser vorgelegt und die Diazotierung eingetragen, wobei man durch Zugabe von Natriumhydrogencarbonat oder Natriumcarbonat einen neutalen pH-Bereich einhält. Nach beendeter Kupplung wird die wässrige Kupplungsbrühe gegebenenfalls mit den für Reaktivfarbstoffe üblichen Stellmitteln (anionische Dispergiermittel, Mononatrium-Dinatrium-phosphat-Puffer, Entstaubungsmittel) versetzt und direkt sprühgetrocknet.

Der Farbstoff enspricht der Formel

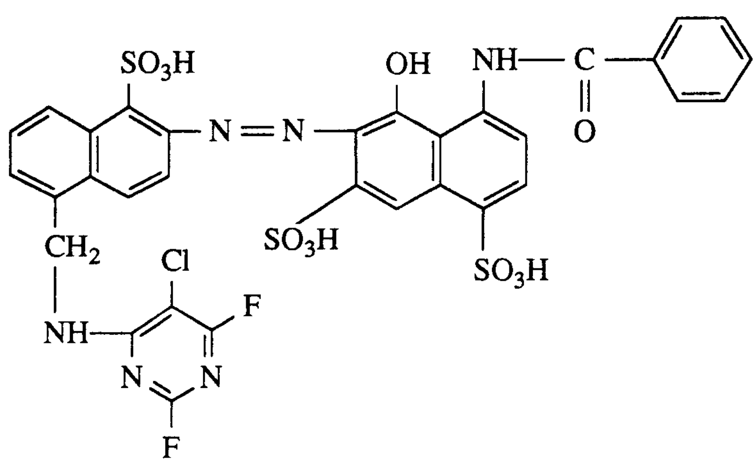

Der Farbstoff ist aus der DE-A 2 232 541 (Beispiel 3) bekannt.

Verwendet man anstelle der 1-Hydroxy-8-benzoylamino-naphthalin-3,5-disulfonsäure die 1-Hydroxy-8-benzoylamino-naphthalin-3,6-disulfonsäure, so erhält man den ensprechenden Farbstoff gleichfalls in hoher Ausbeute.

Beispiel 2

Das nach Beispiel 1, Absatz 1, erhaltene Acylierungsgemisch wird auf pH 7,5 gestellt und auf ca. 55-60 °C erwärmt, wobei das Acylierungsprodukt in Lösung geht. Man setzt dann 1,0 Mol Natriumnitrit in Form einer 30 %igen wäßrigen Lösung zu und läßt das Gemisch in eine Vorlage aus Wasser, 120 g des in

Beispiel 1 verwendeten anionischen Dispergiermittels und Salzsäure einlaufen, wobei man mit Eis die Temperatur zwischen 10 und 20°C hält. Die Kupplung auf 1-Hydroxy-8-benzoyl-amino-naphthalin-3,5-disulfonsäure erfolgt in der in Beispiel 1 beschriebenen Weise. Nach beendeter Kupplung wird das Kupplungsgemisch gegebenenfalls nach einer Aufkonzentrierung durch Druckpermeation direkt sprühgetrocknet.

Beispiel 3

252 g 2-Amino-5-aminomethyl-naphthalin-1-sulfonsäure (1,0 Mol; liegt als Betain vor) werden in 1 800 ml Wasser durch Zugabe von 1 Mol Natronlauge gelöst. In einer Vorlage werden in 400 ml Wasser 38 g Borsäure (0,61 Mol) mit 0,38 mol Natronlauge bei pH 9,5 gelöst. Anschließend fügt man 1 kg Eis hinzu. In diese Vorlage werden zunächst 10 g 2,4,6-Trifluor-5-chlor-pyrimidin und dann gleichzeitig unter guten Rühren 170 g 2,4,6-Trifluor-5-chlor-pyrimidin und die alkalische Lösung der 2-Amino-5-aminomethyl-naphthalin-1-sulfonsäure in 20 Minuten eindosiert. Durch Einwerfen von Eis hält man die Temperatur bei 0°C. Zur Einhaltung des pH-Bereiches von 9,5 (9,3 bis 9,7) müssen während der Acylierung ca. 0,9 Mol Natronlauge zugeführt werden. Man rührt noch ca. 1 Stunde nach, wobei der pH noch geringfügig korrigiert werden muß. Das Kondensationsprodukt ist zum Teil ausgefallen. Volumen 6 l.

Beispiel 4

252 g 2-Amino-5-aminomethyl-naphthalin-1-sulfonsäure (1,0 Mol; liegt als Betain vor) werden in 1 800 ml Wasser durch Zugabe von 1 Mol Natronlauge gelöst. In einer Vorlage werden in 400 ml Wasser 38 g Borsäure (0,61 Mol) mit 0,38 mol Natronlauge bei pH 9,5 gelöst. Anschließend gibt man ca. 200 g Eis hinzu um die Temperaturauf 15°C zu bringen.

In diese Vorlage werden gleichzeitig unter gutem Rühren 151 g 4,6-Difluor-5-chlor-pyrimidin und die alkalische Lösung der 2-Amino-5-aminomethyl-naphthalin-1-sulfonsäure in 20 Minuten eindosiert. Durch Einwerfen von Eis hält man die Temperaturbei 15°C. Zur Einhaltung des pH-Bereiches von 9,5(9,3 bis 9,7) müssen während der Acylierung ca. 0,9 Mol Natronlauge zugeführt werden. Man rührt noch ca. 1 Stunde nach, wobei der pH noch geringfügig korrigiert werden muß. Das Kondensationsprodukt ist zum Teil ausgefallen. Volumen 5 l.

Diazotierung, Kupplung und Isolierung wird nach den Angaben von Beispiel 1 aufgeführt. Der resultierende Farbstoff entspricht der Formel

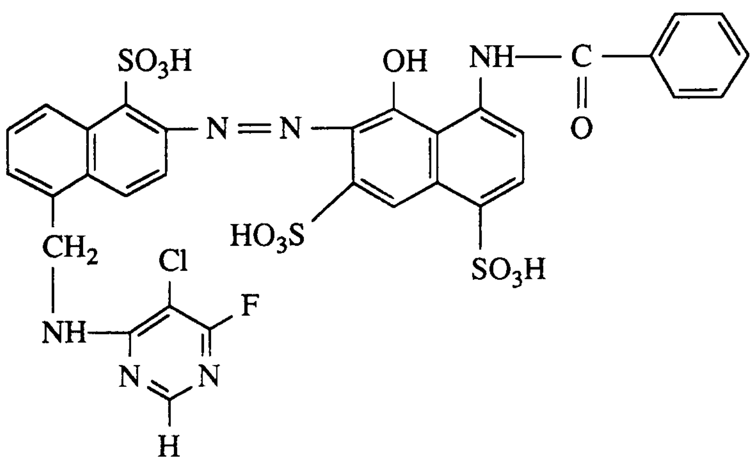

Beispiel 5

Acylierung

252 g 2-Amino-5-aminomethyl-naphthalin-1-sulfonsäure (1,0 Mol; liegt als Betain vor) werden in 1 800 ml Wasser durch Zugabe von 1 Mol Natronlauge gelöst. In einer Vorlage werden in 400 ml Wasser 38 g Borsäure (0,61 Mol) mit 0,38 mol Natronlauge bei pH 9,5 gelöst. Anschließend fügt man 1 kg Eis hinzu. In diese Vorlage werden gleichzeitig unter gutem Rühren die aus 1,05 mol o-Sulfanilsäure und 1,1 Mol 2,4,6-Trifluortriazin nach den Angaben von Beispiel 2 der Deutschen Offenlegungsschrift 2 556 640 bereitete eiskalte Lösung des Monokondensationsproduktes und die alkalische Lösung der 2-Amino-5-aminomethyl-naphthalin-1-sulfonsäure in 20 Minuten eindosiert. Durch Einwerfen von Eis hält man die Temperatur bei 0°C. Zur Einhaltung des pH-Bereiches von 9,5 (9,3 bis 9,7) müssen während der Acylierung ca. 0,9 Mol Natronlauge zugeführt werden. Man rührt noch ca. 1 Stunde nach, wobei der pH noch geringfügig korrigiert werden muß. Das Kondensationsprodukt ist gelöst.

Diazotierung, Kupplung Isolierung

Diese Lösung wird mit 100 g eines anionischen Dispergiermittels (Na-Salz des Kondensationsproduktes aus Naphthalinsulfonsäure und Formaldehyd) versetzt und dann in bekannter Weise bei 0-5°C direkt diazotiert. Die Menge der Kupplungskomponente richtet sich nach dem Nitritverbrauch bei der direkten Diazotierung. Diese Menge an 1-Hydroxy-8-benzoylamino-naphthalin-3,5-disulfonsäure wird in ca. 1 500 ml Wasser vorgelegt und die Diazotierung eingetragen, wobei man durch Zugabe von Natriumhydrogencarbonat oder Natriumcarbonat einen neutalen pH-Bereich einhält. Nach beendeter Kupplung wird die wässrige Kupplungsbrühe, gegebenenfalls mit den für Reaktivfarbstoffe üblichen Stellmitteln (anionische Dispergiermittel, Mononatrium/(Dinatrium-phosphat-Puffer, Entstaubungsmittel) versetzt und direkt sprühgetrocknet.

Der Farbstoff entspricht der Formel

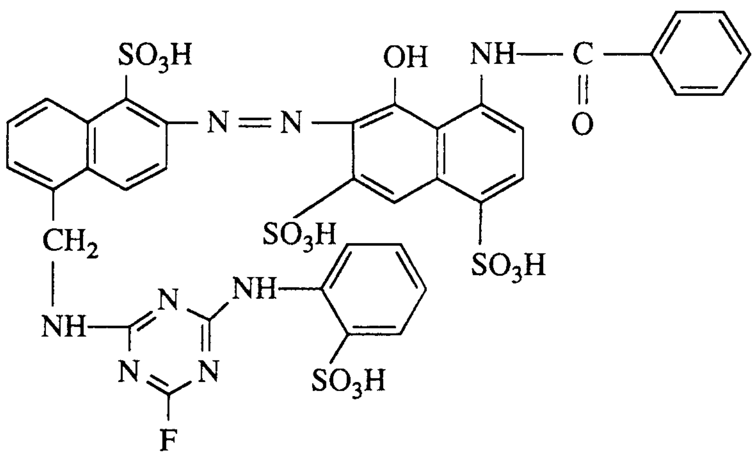

Der Farbstoff ist aus der DE-A 2 847 173 (Beispiel 1) bekannt.

Verwendet man anstelle der 1-Hydroxy-8-benzoylamino-naphthalin-3,5-disulfonsäure die 1-Hydroxy-8-benzoylamino-naphthalin-3,6-disulfonsäure, so erhält man den entsprechenden Farbstoff gleichfalls in hoher Ausbeute.

**Patentansprüche**

**1.** Verfahren zur Herstellung von Verbindungen der Formel

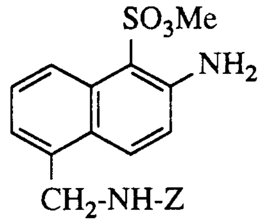

(1),

worin

Z = faserreaktiver Rest einer Reaktivkomponente, der mindestens ein reaktives Fluoratom enthält, und

Me = Kation, insbesondere Li, Na, K, H dadurch gekennzeichnet, daß man eine fluorhaltige Reaktivkomponente der Formel

Z-F (2) (F = Fluor)

und eine wässrige Lösung eines Salzes, insbesondere eines Alkalisalzes, der 2-Amino-5-amino-methyl-naphthalin-1-sulfonsäure (3) in einen Reaktor dosiert, wobei die Reaktivkomponente (2) und die Sulfonsäure (3) gleichzeitig in den Reaktor unter Reaktion eindosiert werden.

**2.** Verfahren gemäß Anspruch 1, dadurch gekennzeichnet, daß Z ein faserreaktiver Rest aus der Pyrimidin- oder Aminotriazinreihe ist.

**3.** Verfahren nach wenigstens einem der vorhergehenden Ansprüche, dadurch gekennzeichnet, daß die Reaktanten im Molverhältnis von etwa 1:1 bei Temperaturen von -5 °C bis 50 °C und in einem pH-Bereich von 8 bis 11 gleichzeitig zusammendosiert werden.

**4.** Verfahren nach wenigstens einem der vorhergehenden Ansprüche, dadurch gekennzeichnet, daß die Reaktivkomponente (2) mit einem Vorlauf von 1 bis 20 % bezogen auf die Gesamtmenge von (2) vorgelegt wird und der Rest von (2) gleichzeitig mit der Lösung von (3) zudosiert wird.

**5.** Verfahren nach wenigstens einem der vorhergehenden Ansprüche, dadurch gekennzeichnet, daß die Reaktionslösung als Puffer ein Alkaliborat enthält.

**6.** Verfahren nach wenigstens einem der vorhergehenden Ansprüche, dadurch gekennzeichnet, daß Verbindung (1) folgender Formel entspricht

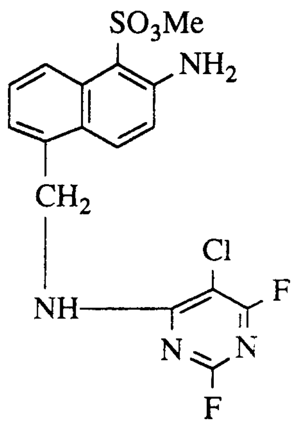

(4).

**7.** Verfahren zur Herstellung von Farbstoffen der Formel

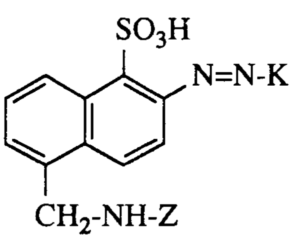

(5),

worin

Z die in Anspruch 1 angegebene Bedeutung hat und

K für den Rest einer Kupplungskomponente HK steht,

dadurch gekennzeichnet, daß man die nach wenigstens einem der vorhergehenden Ansprüche hergestellte Verbindung der Formel (1) diazotiert und anschließend mit der Kupplungskomponente HK kuppelt.

8. Verfahren nach Anspruch 7, dadurch gekennzeichnet, daß man die Diazotierung in Gegenwart eines anionischen Dispergiermittels ausführt.

9. Verfahren nach wenigstens einem der Ansprüche 7 und 8, dadurch gekennzeichnet, daß man die nach der Kupplung anfallende Farbstoff-Lösung oder Suspension gegebenenfalls nach Zusatz der für Reaktivfarbstoffe üblichen Stellmittel direkt zur Trockne bringt.

10. Verfahren zur Herstellung von Farbstoffen nach wenigstens einem der Ansprüche 7 bis 9, dadurch gekennzeichnet, daß K für den Rest

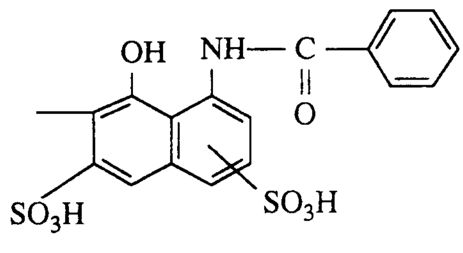

und Z für 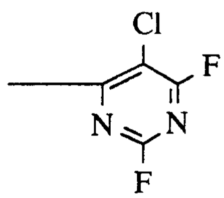 steht.

Europäisches Patentamt

# EUROPÄISCHER RECHERCHENBERICHT

Nummer der Anmeldung

EP 91 12 2367

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | FR-A-2 367 755 (CIBA-GEIGY)<br>* Ansprüche 1,3,10 *<br>--- | 1,2,3,6,7,10 | C 07 D 239/42<br>C 07 D 251/44<br>C 09 B 62/028 |
| Y | EP-A-0 172 790 (CIBA-GEIGY)<br>* Ansprüche 1,2,3,7,8,9,10,12 *<br>--- | 1,2,3,6,7,10 | |
| Y | EP-A-0 053 097 (CIBA-GEIGY)<br>* Anspruch 8; Seite 5, Zeilen 24-29 *<br>--- | 1,2,7 | |
| Y | EP-A-0 043 927 (BAYER)<br>* Ansprüche 1,2,7,8,9; Seite 4, Zeilen 3-13 *<br>----- | 1,2,6,10 | |

RECHERCHIERTE SACHGEBIETE (Int. Cl.5)

C 09 B
C 07 D

Der vorliegende Recherchenbericht wurde für alle Patentansprüche erstellt

| Recherchenort | Abschlußdatum der Recherche | Prüfer |
|---|---|---|
| DEN HAAG | 27-02-1992 | GINESTET M.E.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)